# EUROPEAN PATENT SPECIFICATION

(11) **EP 2 349 340 B1**
(45) Date of publication and mention of the grant of the patent: **30.11.2016**
(21) Application number: 09821149.3
(22) Date of filing: 14.10.2009
(51) Int. Cl.: A61P 35/00, A61P 31/10, A61K 9/19, A61K 9/08, A61K 31/09, A61K 47/48, B82Y 5/00, C08L 5/16, C08B 37/00

(54) **STABLE AQUEOUS FORMULATIONS OF WATER INSOLUBLE OR POORLY SOLUBLE DRUGS**
STABILE WÄSSRIGE FORMULIERUNGEN VON WASSERUNLÖSLICHEN BZW. SCHLECHT LÖSLICHEN ARZNEIMITTELN
FORMULATIONS AQUEUSES STABLES DE MEDICAMENTS INSOLUBLES OU PEU SOLUBLES DANS L'EAU

(30) Priority: 15.10.2008 US 105599 P
(43) Date of publication of application: 03.08.2011
(73) Proprietor: Erimos Pharmaceuticals LLC, Houston, TX 77036 (US)
(72) Inventor: LO DUCA BLOMBERG, Jessica, Andrea, Raleigh NC 27615 (US); CHILTON, Anthony, Stephen, Alpharetta GA 30004 (US); INGALLINERA, Thomas, Samuel, Charleston SC 29412 (US)
(74) Representative: Wills, Andrew Jonathan
(86) International application number: PCT/US2009/060581
(87) International publication number: WO 2010/045281

(56) References cited:
- EP-A1- 0 490 193
- WO-A2-01/80828
- WO-A2-2006/014669
- WO-A2-2006/081363
- WO-A2-2007/101111
- WO-A2-2007/101111
- US-A- 5 376 645
- US-A1- 2008 096 967

## Description

### BACKGROUND OF THE INVENTION

The pharmaceutical arts face very significant challenges in delivering water insoluble or poorly soluble drugs into systemic circulation and from there to the target tissue. Dissolving water insoluble or poorly soluble agents into aqueous solutions appropriate for human use (e.g., oral, topical application, intravenous injection, intramuscular injection, and subcutaneous injection) represents a major technological hurdle for pharmaceutical delivery systems. Generally the solubility problem is addressed by using co-solvent systems. Co-solvent systems unfortunately may have increased side effects due to the quantity of the co-solvents administered. Additionally, they require more analytical testing to determine whether the additional co-solvent excipients leach or extract components from the IV tubing sets.

This application relates to compositions of water insoluble or poorly soluble compounds including but not limited to tetra-o-methyl *NDGA,* also known as terameprocol or also called EM-1421, for administration to animals and humans for treatment of diseases, for example, cancer, psoriasis or other proliferative or inflammatory diseases, viral diseases metabolic diseases such as diabetes or neuronal diseases including neurodegenerative diseases such as Alzheimer's disease, stroke, amyotrophic lateral sclerosis and Parkinson's disease.

Terameprocol is synthesized from meso-nordihydroguaiaretic acid (*m*-NDGA) by dimethyl sulfate in the presence of base. The product is isolated and crystallized to give the purified terameprocol product used in the final drug product. Terameprocol is a white to off-white solid (CAS# 24150-24-1). The chemical formula is C₂₂H₃₀O₄ and chemical structure:

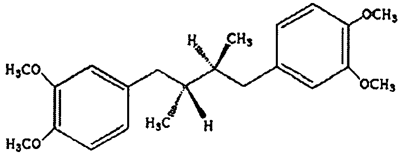

Terameprocol is a small molecule with a molecular weight of 358.5 g/mol; it is hydrophobic, and non-ionic. Due to the properties of the methoxy groups (-O-CH₃) substituted on the aromatic rings, the compound is extremely stable and does not degrade when subjected to base, acid, heat, light, oxidizer, and water.

Terameprocol is an extremely difficult drug to formulate because it is effectively insoluble in all of the commonly used aqueous media used for intravenous delivery of pharmaceuticals. Terameprocol's solubility in an aqueous solvent, at all pH's in water, 0.9% saline and 5% dextrose is < 0.01 mg/mL, a level below the limit of quantitation of the RP-HPLC assay used for evaluation. In order to develop a formulation suitable for clinical use, it is preferred that the solubility of the drug be equal to or exceed 5 mg/mL. At concentrations below this limit, large volumes of solvent are required to deliver the drug. The extremely low solubility of terameprocol presents major formulation challenges using conventional formulation techniques such as the use of organic solvents, surfactants or extremes of pH. These conventional formulation approaches would require the administration of large concentrations of co-solvents and may render the solution viscous and/or difficult to handle during reconstitution in the bedside or pharmacy. Additionally, co-solvents known in the art have been linked to adverse reactions in patients or to precipitation of drug in the bloodstream. The use of conventional approaches have met with limited success when formulating terameprocol for intravenous parenteral administration.

Cylodextrins are water soluble sugar oligomers. Many different cyclodextrins exist and are distinguished from each other by the number of glucopyranose units. The most common cyclodextrins are composed of six, seven or eight alpha-D- glucose units (referred to respectively as α, β and γ cyclodextrins). It is known in the art that cyclodextrins may be used as solubilizing agents for improving the aqueous solubility (i.e. preparation of aqueous base formulations) of certain drugs having poor aqueous solubility). Cyclodextrins form structures having cavities which are hydrophilic on the outside and lipophilic on the inside. The number of glucose units determines the size of the cavity. The hydrophilic exterior gives cyclodextrins their solubility in aqueous solutions while the lipophilic interior or cavity provides an environment which is often attractive to other hydrophobic molecules. By sequestering the drug in the hydrophobic core, it is solubilized with the cyclodextrin molecule so forming an aqueous soluble complex. Cyclodextrins may take up the entirety of a molecule or only a part thereof into the cavity. The stability of the resulting complex depends on how well the drug molecule fits into the cyclodextrin cavity.

There is still a high degree of unpredictability with regards to whether cyclodextrins will improve the solubility of a particular drug. Known methods for preparation of cyclodextrin inclusion complexes require that the drug to be included must be solvated prior to inclusion. With poorly water soluble compounds, a water miscible co-solvent such as alcohol, polyethylene glycols, propylene glycol and/or surfactants have been used to facilitate solvation. The use of co-solvents, particularly at high concentrations may create other problems. For example, while previous experiments were successful in facilitating the creation of a cyclodextrin terameprocol inclusion complex using polyethylene glycol 300, the resulting formulation was linked to adverse reactions in renal impaired patients when administered at high doses in a clinical safety study.

Attempts to omit the co-solvent and directly mix terameprocol with cyclodextrin in water to form the drug-cyclodextrin complexes in the absence of another solvent resulted in unacceptably low concentrations through precipitation of the drug, decomplexing, or failure to ever form a complex.

Formulation of insoluble or poorly water soluble drugs has also been attempted through fusion of the drug and a carrier by combining them at temperatures at or above their melting points. Components are first blended and melted in a suitable mixer and the resulting mixture cooled rapidly to produce a powder. This process unfortunately often leads to unacceptable degradation of the drug as a result of the high temperatures required to melt the drug and the carrier. This would not lead to an acceptable dosage form due to lack of water solubility upon reconstitution.

Meso-nordihydroguaiaretic acid (*"NDGA"*) has been tested for therapeutic applications in in-vivo therapeutic models. For example, Jordan et al. in U.S. Pat. No. 5,008,294 described the effect of *NDGA* on human mammary carcinoma, MX-1, which was implanted subcutaneously into mice on day 0 (Example 2). Mice that developed tumors were injected with various doses of *NDGA* on day 1, in a single intratumor injection. The solubilizing solvent for the *NDGA* in this example was not disclosed. In another example, a stock solution of 10.sup.-2 M *NDGA* in 4 mL of DMSO (i.e., dimethyl sulfoxide) and 6 mL distilled water was used for in vitro testing on cells (Example 5).

Huang et al. tested the therapeutic application of certain derivatives of *NDGA* (i.e., *"NDGA* derivatives"), as described in U.S. Pat. No. 6,214,874. In one example, an *NDGA* derivative was dissolved in DMSO (Example 5).

The use of DMSO for administration into humans has been controversial. Further, use of DMSO has been associated with undesirable side effects such as sedation, headache, nausea, dizziness, burning or aching eyes and noticeable breath odor. (See, for example, Brobyn, R. D., "The human toxicology of dimethyl sulfoxide," Ann. N.Y. Acad. Sci. 243: 497-506, Jan. 27, 1975.) If the catecholic butanes, including *NDGA or NDGA* derivatives (collectively, *"NDGA* compounds") are to be useful as therapeutics for humans and other animals, for example, as described in PCT/US2004/016117, published Dec. 29, 2004 as International Publication No. WO 04/112696, it would be highly desirable to develop new formulations, other than formulations containing DMSO, to solubilize such catecholic butanes, including the *NDGA* compounds and its derivatives, for example, M4N. Furthermore, these formulations should be safe and well tolerated but also stable, with minimal side effects upon administration. To be effective, the formulations for these compounds should effect delivery of an efficacious amount of the unbound active compound to reach the desired target tissues. The present invention provides these desirable benefits.

In a prior application, WO 2006/081364 filed on January 27, 2006, the present inventors working with others developed certain formulations of terameprocol using co-solvent systems to solubilize catecholic butanes.

### BRIEF SUMMARY OF THE INVENTION

The present invention provides for a formulation as described herein for solubilizing terameprocol in cyclodextrins with limited or no use of co-solvents and a novel process for making the formulation.

The objective of the present invention is to provide formulation approaches for solubilizing terameprocol in an aqueous formulation suitable for intravenous injection.

Complexation occurs by melting terameprocol under conditions that do not induce significant degradation whereby the liquefied drug will then be driven into the cyclodextrin cavity that is in solution, thus forming a stable aqueous based formulation.

It is an objective of this invention to solubilize terameprocol, where such formulations do not contain DMSO or alike and are suitable for injection into animals.

It is another objective to provide formulations as above that are safe and have few adverse side effects when administered to animals, including humans.

It is another objective to provide formulations as one or more of the foregoing that have a commercially viable period of stability.

It is a further objective to provide formulations as one or more of the foregoing that can be administered parenterally to animals and humans.

It is another one of the objectives to provide for formulations as one or more of the foregoing that have a therapeutically useful half-life of the active drug in circulation following administration.

In accordance to the foregoing one or more objectives of the invention, there is provided embodiments of the present invention as follows:

A formulation comprising a cyclodextrin and terameprocol with a sufficient amount of water to solubilize the cyclodextrin, and the formulation is obtainable by a method wherein the terameprocol is driven into the cyclodextrin by melting the terameprocol and said solubilized cyclodextrin is heated to a temperature which is above the melting point of terameprocol, but below the decomposition point of terameprocol Described herein is the formulation of the invention for use in a method of treatment of a disease in a subject comprising: (a) providing the composition of the present invention; and (b) administering the composition by injecting the composition into the subject, wherein the composition comprises an effective amount of the API.

### BRIEF DESCRIPTION OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention is not limited to the embodiments shown.

### IN THE DRAWINGS:

Figure 1 is an LC-MS-MS Scan of an Inclusion Complex Utilizing a Co-solvent.
Figure 2 shows Terameprocol (EM-1421) solubility in aqueous hydroxypropyl betacyclodextrin (HPβCD) after 48hrs (high concentration).
Figure 3 shows Terameprocol (EM-1421) solubility in aqueous HPβCD after 48hrs (low concentration).
Figure 4 shows a Hypothetical Illustration of Terameprocol Inclusion Complexes with Cyclodextrin.
Figure 5 shows the Manufacturing Process Flow Chart for the Drug-HPβCD inclusion complex.
Figure 6 shows microscopic images of in-process samples of the terameprocol formulation below the melting point of the drug (presence of crystals).

### DETAILED DESCRIPTION OF THE PREFERRED EMBODIMENTS

compositions for use in The present invention provides for compositions, and compositions for use in methods for treatment of diseases, including proliferative diseases such as cancer and psoriasis, hypertension, obesity, type I or type II diabetes, central nervous system diseases or neurodegenerative diseases including, without limitation, pain, Alzheimer's disease, amyotrophic lateral sclerosis, Parkinson's disease, dementia, stroke, and inflammatory disease, premalignant neoplasia or dysplasia, infection including viral infections such as human immunodeficiency viruses ("HIV"), human T-cell lymphotropic virus ("HTLV"), human papilloma virus ("HPV"), herpes simplex viruses ("HSV"), hepatitis B virus ("HBV"), Epstein-Barr virus ("EBV"), Varicella-zoster, adenovirus, parvovirus, Jakob Creutzfeldt virus ("JC virus") or others.

The present invention provides compositions of terameprocol having a melting point below the melting point of a cyclodextrin The drug is not degraded by heating it to a temperature at least as low as its melting point in the presence of water. In particular the present invention is surprisingly effective at solubilizing terameprocol without the use of co-solvents or solubilizing agents known to have toxicities.

In addition, the composition may be used together with suitable materials for injection or infusion of the composition into an animal, such as intravenous ("IV") tubing, that is compatible for delivery of the present formulations. Suitable tubing include those made of polymers such as polytetrafluoroethylene ("PTFE") alone or in combination but not limited to with a fluoroelastomer such as CHEM-Sure (Barnant Company), polyethylene, polypropylene, fluorinated ethylene propylene ("FEP"), Teflon® and platinum cured silicone.

The present invention can be more clearly understood in light of the following definitions, which are used with other terms as defined elsewhere herein:
The term "about" as used herein in reference to a concentration or dose means the specified number up to .+-.10% to 20%.

The term "active pharmaceutical ingredient," "API" or reference to the "compounds" as used herein means any of the catecholic butanes of Formula I or the *NDGA* compounds, such as the *NDGA* derivatives, present in the pharmaceutical compositions herein, that satisfy said parameters.

The "buffer" suitable for use herein includes any buffer conventional in the art, such as, for example, Tris, phosphate, imidazole, and bicarbonate.

A "carrier" as used herein refers to a non-toxic solid, semisolid or liquid filler, diluent, vehicle, excipient, solubilizing agent, encapsulating material or formulation auxiliary of any conventional type, and encompasses all of the components of the composition other than the active pharmaceutical ingredient. The carrier may contain additional agents such as wetting or emulsifying agents, or pH buffering agents. Other materials such as anti-oxidants, humectants, viscosity stabilizers, and similar agents may be added as necessary.

A "cyclodextrin" as used herein means an unmodified cyclodextrin or a modified cyclodextrin, and includes with out limitation α-cyclodextrin, β-cyclodextrin, γ-cyclodextrin and any modified cyclodextrins containing modifications thereto, such as HPβCD or SBEβCD. Cyclodextrin typically has 6 (α-cyclodextrin), 7 (β-cyclodextrin), and 8 (γ-cyclodextrin) sugars, up to three substitutions per sugar, and 0 to 24 primary substitutions are therefore possible (primary substitutions are defined as substitutions connected directly to the cyclodextrin ring). The modified or unmodified cyclodextrins used in the present invention may have any appropriate number and location of primary substitutions or other modifications.

A "derivative" of *NDGA* as used herein means an *"NDGA* derivative" (see below).

The term "disease" as used herein includes all diseases, conditions, infections, syndromes or disorders for which the application of the present composition produces a therapeutic effect. Such "disease" includes, for example without limitation, cancer, psoriasis and other proliferative diseases, inflammatory disorders including rheumatoid arthritis, osteoarthritis, ulcerative colitis, Crohn's disease, atherosclerosis, chronic obstructive pulmonary disease ("COPD"), hypertension, obesity, diabetes, pain, stroke and/or other neuronal disorders or neurodegenerative diseases or conditions, including Alzheimer's disease, Parkinson's disease, multiple sclerosis, amyotrophic lateral sclerosis ("ALS") and premalignant conditions such as intraepithelial neoplasia or dysplasia, and infectious diseases.

The term "water insoluble pharmaceutical" or "poorly soluble" as used herein means a drug having solubility in water below that required to produce a clinically acceptable therapeutic dose.

"M₄N", "EM-1421" or "terameprocol" as used herein means tetra-o-methyl nordihydroguaiaretic acid or tetra-o-methyl NDGA.

*"NDGA"* as used herein means nordihydroguaiaretic acid.

*"NDGA* compound" as used herein means singly or collectively *NDGA* and/or any one or more *of the NDGA* derivatives.

As used herein, "percent," "percentage" or the symbol "%" means the percent of the component indicated in the composition based on the amount of the carrier present in the composition, on a weight/weight (w/w), weight/volume (w/v) or volume/volume (v/v) concentration, as indicated with respect to any particular component, all based on the amount of the carrier present in the composition. Thus, different types of carriers may be present in an amount of up to 100% as indicated, which does not preclude the presence of the API, the amount of which may be indicated as a % or as a certain number of mg present in the composition or a certain number of mg/mL present, where the % or mg/mL is based on the amount of the total carrier present in the composition. Certain types of carriers may be present in combination to make up 100% of the carrier.

A "pharmaceutically acceptable carrier" as used herein is non-toxic or have certain toxic attributes that are not dose limiting to achieve therapeutic advantages to recipients at the dosages and concentrations required and is compatible with other ingredients of the formulation. For example, the carrier for a formulation containing the present catecholic butane, *NDGA* compounds or *NDGA* derivatives preferably does not include oxidizing agents and other compounds that are known to be deleterious to such. A pharmaceutically acceptable carrier comprises a solubilizing agent.

The term "pharmaceutically acceptable excipient," includes vehicles, adjuvants, or diluents or other auxiliary substances, such as those conventional in the art, which are readily available to the public, and which are non-toxic or have acceptable toxicities to recipients at the dosages and concentrations employed, and is compatible with other ingredients of the formulation. For example, pharmaceutically acceptable auxiliary substances include pH adjusting and buffering agents, tonicity adjusting agents, stabilizers, wetting agents and the like.

The term "solubilizing agent" as used herein means a composition in which solubilizes a pharmaceutical agent. A solubilizing agent may also be a carrier.

The terms "subject," and "patient," are used interchangeably herein to refer to an animal being treated with the present compositions, including, but not limited to, simians, humans, felines, canines, equines, bovines, porcines, ovines, caprines, mammalian farm animals, mammalian sport animals, and mammalian pets.

As used herein, the terms "treatment," "treating," and the like, refer to obtaining a desired pharmacologic and/or physiologic effect. The effect may be prophylactic in terms of completely or partially preventing a condition or disease or symptom thereof and/or may be therapeutic in terms of a partial or complete cure for a condition or disease and/or adverse affect attributable to the condition or disease. "Treatment," thus, for example, covers any treatment of a condition or disease in a mammal, particularly in a human, and includes: (a) preventing the condition or disease from occurring in a subject which may be predisposed to the condition or disease but has not yet been diagnosed as having it; (b) inhibiting the condition or disease, such as, arresting its development; and (c) relieving, alleviating or ameliorating the condition or disease, such as, for example, causing regression of the condition or disease.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

It must be noted that as used herein, the singular forms "a", "an", and "the" include plural referents unless the context clearly dictates otherwise. Thus, for example, reference to "a compound" includes a plurality of such compounds and reference to "the *NDGA* compound" includes reference to one or more *NDGA* compounds, such as *NDGA* derivatives, and equivalents thereof known to those skilled in the art.

### Preparation of Catecholic Butanes

The catecholic butanes can be prepared by any method know in the art. For example, such compounds can be made as described in U.S. Pat. No. 5,008,294.

### Preparation of the Therapeutic Compositions

The present invention provides compositions, including pharmaceutical compositions, comprising the catecholic butanes, including the *NDGA* compounds, such as the *NDGA* derivatives, as active pharmaceutical ingredients ("API"), and pharmaceutically acceptable carriers or excipients. Typically, the compositions of the instant invention will contain from less than about 0.1% (w/w) up to about 20% (w/w) of the active pharmaceutical ingredient or API, that is, the catecholic butanes, including the *NDGA* compounds and *NDGA* derivatives herein; optionally, the present invention will contain about 0.2% (w/w) to about 8% (w/w) of the API.

The pharmaceutically acceptable carrier or excipient may additionally contain a diluent.

The present invention provides compositions of water insoluble or poorly soluble compounds in a cyclodextrin, which includes modified cyclodextrins. The cyclodextrins herein may be α-cyclodextrin, β -cyclodextrin, γ-cyclodextrin, and the modified cyclodextrins may include HPβCD and SBEβCD, for example. In one embodiment, the present composition contains a modified cyclodextrin in a concentration of about 5% to about 80%, or about 10% to about 70%, or about 20% to about 60%, or about 30% to about 50%, all such concentrations being given as a percentage of weight/weight (w/w) concentration.

In a preferred embodiment HPβCD and at least sufficient water to dissolve the cyclodextrin in a vessel. The active agent is then added and mixture is homogenized and heated to a temperature from about 80 °C to about 131 °C until the active agent is solubilized. In a most preferred embodiment the temperature is from about 104 °C to about 115 °C.

In yet another embodiment the pH of the formulation is between 3 and 10, most preferred being pH 7. pH may be adjusted using pharmaceutically accepted agents known in the art for adjusting pH. pH may be adjusted before, during and/or after solubilization of the active agent.

In yet another embodiment the homogenization vessel is pressurized to pressurized from about 0 psig to about 30 psig.

In yet another embodiment, the mixture of HPβCD and active agent are homogenized from about 30 minutes to about 24 hours.

While the present invention most preferably does not contain any type of co-solvent or other excipients to enhance solubility, one of skill in the art will appreciate that such co-solvents or excipients while they are not necessary, can be used without deviating from the spirit of the present invention.

It is to be understood that as long as the catecholic butanes herein are dissolved and remain in solution, one or more of the solubilizers or diluents or excipients herein may be added to such solution to optimize delivery of such to a subject in need of such treatment.

In another embodiment, the invention provides a diluent that is saline or water that is suitable for injection. In one aspect of the invention, when the osmolarity of the pharmaceutical composition is high, water suitable for injection is used as a diluent.

The compositions in liquid form may include a buffer, which is selected according to the desired use of the terameprocol, and may also include other substances appropriate for the intended use. Those skilled in the art can readily select an ppropriate buffer, a wide variety of which are known in the art, suitable for an intended use.

### Therapeutic Methods

The compositions containing terameprocol find use as therapeutic agents or for treatment in subjects in need of such treatment in any number of diseases in which terameprocol can be used.

The present invention provides for methods and compositions for treatment of disease including, for example, proliferative diseases such as benign and malignant cancer, including but not limited to solid tumors, leukemia, brain tumors, myelomas, psoriasis and premalignant conditions and neoplasia, such as intraepithelial neoplasia, or dysplasia. The present invention also provides for treatment of diabetes, including type I and type II diabetes, obesity and complications resulting from such, including cardiovascular diseases, stroke and hypertension. The present invention further provides for treatment of inflammatory diseases including rheumatoid arthritis, osteoarthritis, multiple sclerosis, ulcerative colitis, Crohn's disease, chronic obstructive pulmonary disease (COPD) and other immune system associated diseases. Additionally, the present invention provides for treatment of neurological diseases, including central nervous system diseases and neurodegenerative diseases such as Alzheimer's disease, dementia, amyotrophic lateral sclerosis and Parkinson's disease. In a further embodiment, the present invention provides for treatment of infections, such as viral infections including viruses that require Spl binding for transcription or replication. Examples of such viruses that require Spl binding include: HIV, HTLV, HPV, HSV, HBV, EBV, Varicella-zoster virus, adenovirus, parvovirus and JC virus.

A variety of animal hosts are treatable according to the subject methods, including human and non-human animals. Generally such hosts are "mammals" or "mammalian," where these terms are used broadly to describe organisms which are within the class mammalia, including the orders carnivore (e.g., dogs and cats), rodentia (e.g., guinea pigs, and rats), and other mammals, including cattle, goats, horses, sheep, rabbits, pigs, and primates (e.g., humans, chimpanzees, and monkeys). In many embodiments, the hosts will be humans. Animal models are of interest for experimental investigations, such as providing a model for treatment of mammalian disease. Further, the present invention is applicable to veterinary care as well.

### Formulations, Dosages, and Routes of Administration

In one embodiment of the invention, an effective amount of the present composition is administered to the host, where an "effective amount" means a dosage sufficient to produce a desired result.

The appropriate dose to be administered depends on the subject to be treated, such as the general health of the subject, the age of the subject, the state of the disease or condition, the weight of the subject, the size of the tumor, for example. Generally, about 0.1 mg to about 10 grams or less may be administered to an adult. Typical dosages are within the broad range of about 10 mg of active pharmaceutical ingredient per kg weight of the subject to about 600 mg of active pharmaceutical ingredient per kg weight of the subject. The active agent can be administered in a single or, more typically, multiple doses. Preferred dosages for a given agent are readily determinable by those of skill in the art by a variety of means. Other effective dosages can be readily determined by one of ordinary skill in the art through routine trials establishing dose response curves. The amount of agent will, of course, vary depending upon the particular agent used.

The frequency of administration of the active agent, as with the doses, will be determined by the care giver based on age, weight, disease status, health status and/or patient responsiveness. Thus, the agents may be administered one or more times daily, weekly, monthly or as appropriate as conventionally determined. The agents may be administered intermittently, such as for a period of days, weeks or months, then not again until some time has passed, such as 3 or 6 months, and then administered again for a period of days, weeks, or months.

In pharmaceutical dosage forms, the active agents may be administered alone or in appropriate association, as well as in combination, with other pharmaceutically active agents or therapeutics including other small molecules, antibodies or protein therapeutics. The following methods and excipients are merely exemplary and are in no way limiting.

In addition, if desired, the carrier or excipient may contain minor amounts of auxiliary substances such as pH adjusting and buffering agents, tonicity adjusting agents, stabilizers, wetting agents or emulsifying agents. Actual methods of preparing such dosage forms are known, or will be apparent, to those skilled in the art. See, e.g., Remington's Pharmaceutical Sciences, 20th ed., Mack Publishing Co. Rawlins E A, (1997). The composition or formulation to be administered will, in any event, contain a quantity of the API adequate to achieve the desired state in the subject being treated.

Unit dosage forms for injection or intravenous administration may comprise the API in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of API of the present invention calculated in an amount sufficient to produce the desired effect in association with a pharmaceutically acceptable diluent, carrier or vehicle. The specifications for the novel unit dosage forms of the present invention depend on the particular compound employed and the effect to be achieved, and the pharmacodynamics associated with each compound in the host.

Kits with multiple or unit doses of the active agent are described herein In such kits, in addition to the containers holding the multiple or unit doses of the compositions containing terameprocol will be an informational package insert with instructions describing the use and attendant benefits of the drugs in treating pathological condition of interest. Optionally, an applicator for administration of the present composition is included in each kit. terameprocol

The present injectable compositions can be administered parenterally, including intravenously, intra-arterially, intraperitoneally, subcutaneously and intravesicularly, as appropriate for the disease to be treated and as conventional in the art. While the compositions of the present invention are intended to be administered by injection, they may also be suitable to be administered by other routes, for example by topical, intranasal, inhalation or implantation administration. The Examples set forth below are exemplary in nature and are not to be interpreted as limiting the present invention.

### Example 1. Solubility of Terameprocol in Various Solvents

The solubility of terameprocol was tested in various solvents and dispersed systems. An excess of the solid API (20-50 mg) was shaken with 1 mL of a given solvent overnight (∼16 hr) at room temperature on a gyratory shaker at maximum speed. The samples were filtered through 0.45 µm PVDF filter. The filtrates were diluted to the working linear range (0.06-0.1 mg/mL) of the UV-Vis method appropriately in acetonitrile and analyzed for terameprocol concentration using a UV-Vis spectrophotometer. The equation of the standard curve was y =16.70x + 0.016 with a R² = 0.999 monitored at 281 nm. These results are shown in Table 1 where RT was ∼ 25 °C.

**Table 1: Solubility of terameprocol in various solvents and dispersed systems**

| **Solvent** | **Solubility (mg/mL)** | **Temperature** | **Assay** |
|---|---|---|---|
| Methanol | 5.71 | RT | UV-Vis |
| Ethanol | 6.22 | RT | UV-Vis |
| IPA | 4.30 | RT | UV-Vis |
| Chloroform | >50 | RT | Visual |
| Methylene Chloride | >50 | RT | Visual |
| Soybean Oil | 7.68 | RT | UV-Vis |
| MCT Oil | 13.4 | RT | UV-Vis |
| Intralipid | 1.38 | RT | UV-Vis |
| Tween (5%) | 0.159 | RT | UV-Vis |
| Pluronic F-68 (5%) | <0.095 | RT | UV-Vis |
| Acetonitrile | >50 | RT | Visual |
| HPβCD (30%) | 1.27 | RT | UV-Vis |
| Propylene Glycol | 0.215 | RT | UV-Vis |
| 20% Ethanol/5% Tween 80/10% PEG 300 in water | 0.593 | RT | UV-Vis |
| 20% Ethanol/5% Tween 80 in PEG 300 | 65.5 | 60°C | UV-Vis |
| PEG 400 | 9.38 | RT | UV-Vis |
| PEG 300 | 10.1 | 40°C | UV-Vis |
| PEG 300 | 18.3 | 60°C | UV-Vis |
| PEG 300 | >600 | 95°C | Visual |
| 10% Ethanol in PEG 300 | >400 | 80°C | Visual |

Samples that did not have excess insoluble drug were not scanned for absorbance to determine terameprocol concentration because the solubility values for these samples shown in Table 1 are based on visual evaluation. As shown in the table, Terameprocol did not have any appreciable solubility (<10 mg/mL) in pharmaceutically relevant solvents at room temperature. Solubility in PEG 300 increased with increasing temperature with the value being greater than 600 mg/mL at 95 °C.

Both chloroform and methylene chloride resulted in good solubility of terameprocol suggesting that either a liposomal or emulsion formulation may be viable to achieve the target concentration of 5-20 mg/mL for an intravenous infusion. MCT oil provided better solubility than soybean oil for the API. Although the inherent solubility is lower for the modified cyclodextrin (HPβCD) in compared to the other solvents listed in Table 1, optimizing the formulation process should increase the amount of terameprocol solubilized due to this intrinsic solubility. This is accomplished by putting enough energy into the system to enhance the motility of the API to orientate itself to facilitate entry into the cyclodextrin cavity, enabling equilibrium to be established and altering the energy state of the components. The solvents in Table 1 such as ethanol and oils are undesirable for an intravenous drug product of terameprocol due to potential side effects of these solvents when administered as part of a theoretical effective dose.

It is known that cyclodextrins may be used as solubilizing agents that allow the formulation of certain poorly water-soluble drugs into an aqueous solution. Cyclodextrins are water-soluble cyclic sugar oligomers (oligosaccharides), which differ from one another in the number of glucopyranose units. The most readily available cyclodextrins are composed of six, seven or eight alpha-D-glucose units (α, β and γ respectively). The solubility of a drug may be increased by virtue of the inclusion of the drug in the relatively hydrophobic cavity of the cyclodextrin. However, it is by no means certain that cyclodextrins will improve the apparent solubility of a particular drug. Cyclodextrins are cyclic oligosaccharides with hydroxyl groups on the outer surface and a void cavity in the center. Their outer surface is hydrophilic, and therefore they are usually soluble in water, but the cavity has a lipophilic character. The most common cyclodextrins are α-cyclodextrin, β-cyclodextrin and γ -cyclodextrin, consisting of 6, 7 and 8 alpha-1,4-linked glucose units, respectively. The number of these units determines the size of the cavity.

Cyclodextrins have a hydrophilic exterior (water soluble) and a hydrophobic-like interior, providing a microenvironment for nonpolar molecules or analytes to form an inclusion complex without forming or breaking any noncovalent bonds [Valle (2004)]. Cyclodextrins are capable of forming inclusion complexes with a wide variety of hydrophobic molecules by taking up a whole molecule (a "guest molecule"), or some part of it, into the void cavity. The analytes must be of a certain size and shape as to minimize the steric hindrance and to fit inside the cavity [Rajewski, et al. (1996); Rekharsky, et al. (1998)]. There are multiple driving forces for inclusion complexes to occur. The primary is the release of the water molecules from the cyclodextrin interior upon complexing with a nonpolar molecule, resulting in a more favorable (lower) energy state [Valle (2004); Rekharsky, et al. (1998)]. Other contributing forces could be: increasing the number of hydrogen bond formed with the bulk aqueous surroundings once the water is displaced from the cyclodextrin cavity, increasing the number of van der Waals forces and hydrophobic interactions of the system once inclusion complex forms, and reducing the repulsive forces present under normal conditions which separate the nonpolar analyte from its the polar, aqueous surroundings (i.e. precipitation) [Valle (2004); Rekharsky, et al. (1998)].

The stability of the resulting complex depends on how well the guest molecule fits into the cyclodextrin cavity. The hydrophobic cavity of the cyclodextrin molecule can act as a host and hold a variety of molecules, or guests that include a hydrophobic portion to form a cyclodextrin inclusion complex. Thus if a hydrophobic molecule can reach the cavity of the cyclodextrin it can be retained in the cavity due to the resulting hydrophobic interactions between the guest molecule and the cyclodextrin cavity. The guest molecule needs to acquire sufficient energy to gain the necessary motility in the complexing process to be able to approach the cavity and orient itself into the cavity. The motility can be affected by utilizing a solvent capable of partially dissolving the guest molecule or creating a lipophilic environment that induces a partition mechanism driving the hydrophobic guest molecule to enter the cavity. The bond formed during complexation is not fixed or permanent but a dynamic equilibrium [Szetjli (1998)].

With poorly water-soluble or insoluble compounds and as disclosed in the previous art, water miscible solvents such as alcohol, polyethylene glycols, propylene glycol, and surfactants have been used to facilitate complexation by dissolving the molecule so that it can freely bind with the cyclodextrin cavity. However, the use of these solvents, specifically polyethylene glycol 300 (PEG 300) which could be dose limiting thus limits the target population and doses given in the clinic.

Previous products utilized a co-solvent technique to first dissolve terameprocol in solvents such as PEG 300, so that the terameprocol molecule could form an inclusion complex within the hydrophobic interior of the HPβCD cavity. The sole purpose of utilizing the co-solvent, PEG 300, was to aid in dissolution of terameprocol thereby, increasing the population of terameprocol molecules available to complex.

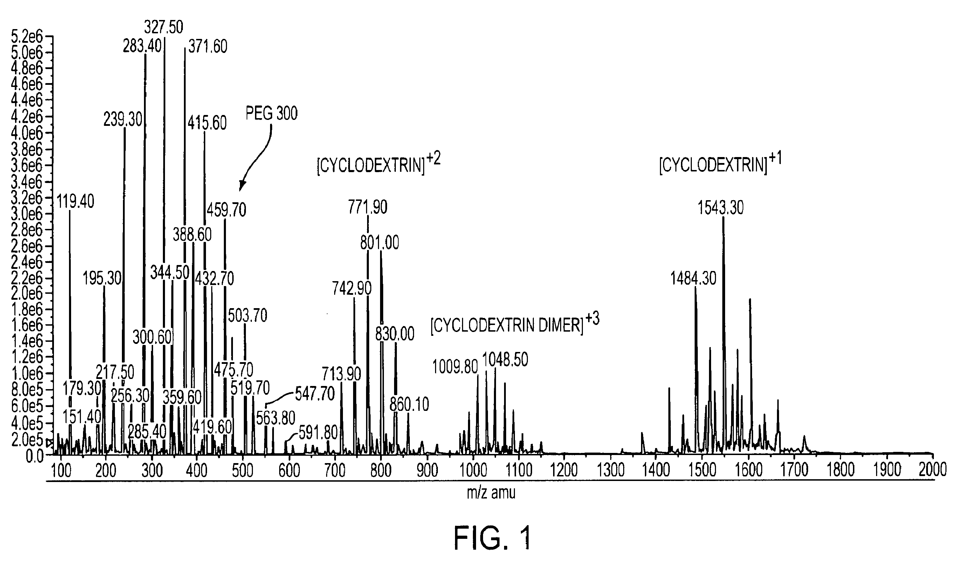

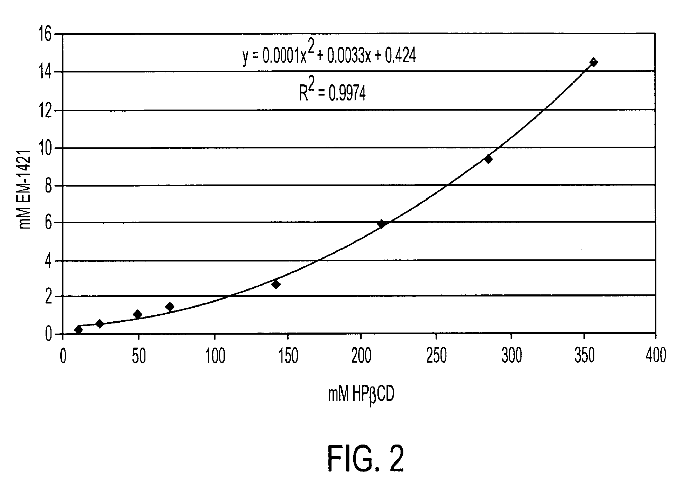

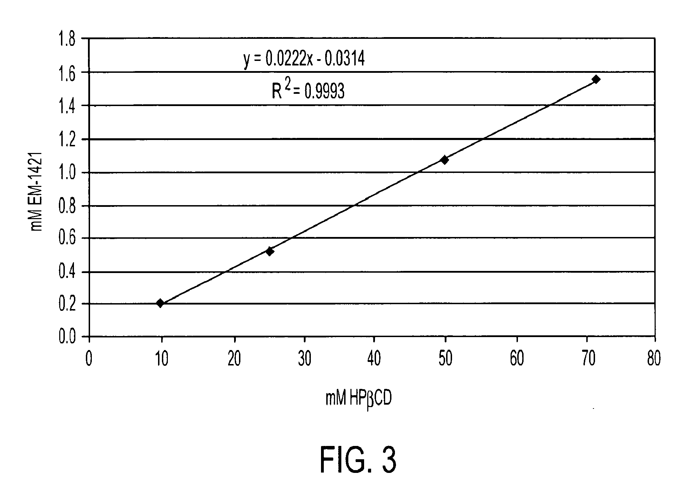

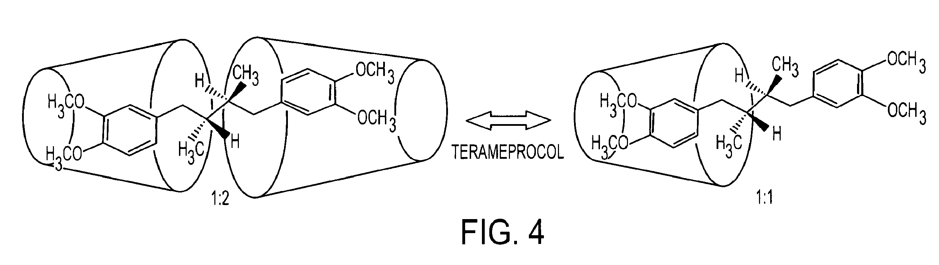

Studies conducted on cyclodextrin formulations of terameprocol indicated that a theoretical conclusion can be drawn that the inclusion complex formed is actually a mix population of a 1:1 (terameprocol:HPβCD) and 1:2 (terameprocol:HPβCD) or higher. Figure 1 shows a cyclodextrin dimer population. This finding, combined with the conclusions illustrated by Figures 2 and 3, that an "Aₚ-type phase-solubility in which the complex is first order with respect to the terameprocol substrate but second or higher order with respect to the HPβCD ligand, infers that, without advance stoichiometric studies, the mix inclusion complex population being, 1:1, 1:2 and/or higher. Figure 4 is a theoretical sketch of the 1:1 and 1:2 complexes that terameprocol forms with HPBCD.

The inventors of the present invention have made the surprising discovery that terameprocol can be formulated in cyclodextrins without the need for elaborate chemical solvent matrices and that lead to increases in the solubility of water insoluble drugs through the formation of a stable inclusion complex. However, mixing the insoluble molecule, terameprocol, with the cyclodextrin inclusion complexes in water without solvent at normal working temperatures all temps below the melting points were observed to produce unacceptable concentrations at and under 7 mg/mL or lead to precipitation of the terameprocol. Details of this will be discussed later.

The present invention yields viable aqueous concentrations of terameprocol without using organic solvents. This is achieved by heating the terameprocol above its melting point with mixing thereby enabling an inclusion complex to form.

The terameprocol/ HPβCD inclusion complex is formed by first dissolving HPβCD in water prior to complexation. The property of HPβCD which is of highest concern is not the stability of melting HPβCD since it is dissolved but the stability of HPβCD during compounding. Based upon the literature provided by Roquette for an HPβCD sold under the product name: Kleptose HPP PF, HPβCD is stable during sterilization at 121°C for 15 minutes and only incurred slight hydrolysis under extreme acidic conditions of pH 2 at the elevated temperature of 60 °C. Coloration of the solution (degradation of sugar rings) is observed when degradation occurs. No significant hydrolysis was observed at pH 4, at 40 °C after 24 hrs [Roquette Fréres (2007)]. Using these proven attributes of HPβCD, degradation is not expected of HPβCD under the processing conditions (described below) used for the present invention and during development. If a color change was observed, it would have been noted in the appearance test and most likely with unstable complex formation.

### Example 2:

Bench-top (10 mL) to 5 L scale development studies were performed to remove PEG 300 from terameprocol/HPβCD formulations. Conclusions from the bench scale experiments were (a) 7 mg/mL is the maximum achievable terameprocol concentration in an IV formulation containing 30-40% HPβCD (b) incorporating the drug at high temperatures (80 °C or greater) is an important step in forming the inclusion complex (c) high mechanical energy must be provided when cooling the complex to approximately ambient condition once formed. There were differences in equipment used at the 10 mL vs. 5 L scale. The 10 mL scale utilized glass beakers with a sonicator for incorporation, while the 5 L experiments were conducted with a 10 L stainless steel jacketed vessel equipped with a recirculating heating bath, overhead air powered blade mixer and an in-line homogenizer.

The 5 L experiments were conducted to (a) explore the acceptable temperature range indicated in 'b' above required for forming terameprocol cyclodextrin complexes, (b) evaluate the stability of terameprocol/ HPβCD upon autoclaving: 15 minutes vs. 30 minutes at 121°C, (c) evaluate the compatibility of terameprocol/ HPβCD with 0.5%(w/v) sodium chloride (NaCl), and (d) develop a process which could be scaled to the next engineering size of 60L. The acceptable temperature required for complexation of terameprocol and cyclodextrin (HPβCD) was determined to be above the melting point of terameprocol (>104°C). The resulting terameprocol/ HPβCD formulation was stable for 4 weeks at room temperature (RT), 4 weeks under refrigerated conditions, and 2 weeks under accelerated conditions (40°C) for all sample types evaluated: non-autoclaved, autoclaved up to 30 minutes, and upon spiking with NaCl as shown in Table 2. The compatibility of terameprocol/ HPβCD with NaCl supports flushing of the IV lines with 0.9% Saline to keep lines patent if required.

**Table 2: Stability of Terameprocol with NaCl and after Autoclaving**

| **Sample** | **Autoclaved** | **Terameprocol (mg/mL)** | **Precipitation** | | |
|---|---|---|---|---|---|
| | | | **RT (4-weeks)** | **2-8 °C (4-weeks)** | **40 °C (2-weeks)** |
| Unspiked | No | 5.80 | No | No | No |
| 0.5% w/v NaCl | No | 5.89 | No | No | No |
| Unspiked | 15 min | 5.76 | No | No | No |
| 0.5% w/v NaCl | 15 min | 6.03 | No | No | No |
| Unspiked | 30 min | 5.97 | No | No | No |
| 0.5% w/v NaCl | 30 min | 5.77 | No | No | No |

The optimized process developed at 5 L was transferred to the 60 L scale.

The 5 L terameprocol formulation process was scaled up to 60 L lots. The 60 L lots were conducted to (a) evaluate the criticality of using an homogenizer to promote complexation of the terameprocol and HPβCD (b) evaluate the stability of the terameprocol/ HPβCD complex upon pH adjustment after compounding using NaOH and/or HCl (c) investigate the stability after autoclaving up to 30 minutes at 121°C of the non pH adjusted, pH adjusted, homogenized, and non-homogenized terameprocol samples (d) optimize the 5 L formulation process to 60 L in order to support a clinical scale-up to 373 L.

It was determined that with proper tank design using an aggressive mixer introduced enough energy into the system where the homogenizer may not be critical for complexation. Samples of terameprocol that had 1) no pH adjustment with and without homogenization and 2) pH adjusted to 7 with no homogenizer and with and without autoclaving were stable at the accelerated condition of 40°C/75%RH for 1 month.

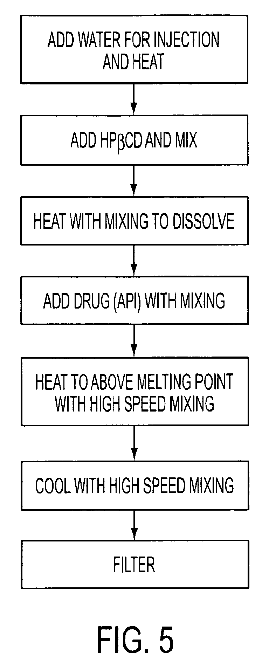

Fundamentally, the same compounding procedure identified at the 60 L scale was implemented at the 373 L (Figure 5). The minor adjustments in the 373 L terameprocol/ HPβCD process were based upon scale and equipment requirements, such as quantity sufficient (QS) of HPβCD solution and defining acceptable temperature.

Composition of the present invention may be formulated wherein said water insoluble or partially soluble drug and water soluble carrier are mixed in a ratio of from about 1:100 to about 1:10 respectively, on a percent weight basis.

One of skill in the art will appreciate that the above formulation can be used in either for either injection or oral administration. It is also contemplated that the above formulation can be lyophilized using methods known in the art and sold in a dry form for reconstitution prior to administration to a patient. Such lyophilized form may offer even greater stability, and reduced costs due to the reduced volume and weight required for shipping and storage.

### Example 3 Determination of Optimum Temperature Requirement

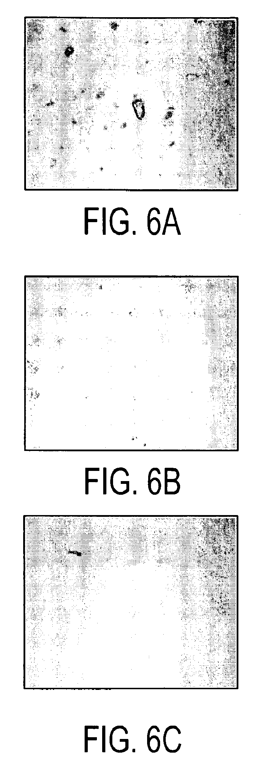

A solution of HPβCD was prepared in a 5 L jacketed vessel and heated to aid in dissolution. Terameprocol was added slowly to the HPβCD solution. The in-line homogenizer was utilized and the temperature was maintained at 40 °C for 2 hours. An in-process sample was taken and the microscopic image is shown in Figure 6-A. There were still numerous terameprocol crystals present. The temperature was increased to 60 °C with homogenization and held for one hour. The microscopic image is shown in Figure 6-B. The number of crystals was reduced from Figure 6 -A; however, the presence of crystals was still evident. The temperature was increased to 80 °C under homogenization for 2 hours. It was noted that due to heating in a closed vessel, the system was slightly pressurized due to the increase in operating temperature. An in-process sample was taken at this point and is shown in Figure 6-C. In compared to A and B, image C showed a significant reduction in crystal formation. The increase in temperature improved the dissolution of terameprocol; however, complete dissolution and complexation did not occur. The experiment was repeated but the system was taken to temperature above the melting point (> 104 °C) with homogenization in the same closed vessel. The vessel was then cooled and samples taken. Stability results were shown previously in Table 2 and no crystal formation was noted immediately or after 4 weeks at presented conditions. It was concluded the optimum temperature requirement was that above the drug's melting point.

### Example 4 Combination Products

It is believed that the formulations of the present invention will facilitate the simultaneous formulation of a water insoluble or poorly soluble drug with another active agent which is either water insoluble itself or water soluble. In pharmaceutical dosage forms, the active agents may be administered alone or in appropriate association, as well in combination with other pharmaceutically active agents or therapeutics including other small molecules, antibodies, or protein therapeutics. Additionally, the API should not significantly degrade when exposed to heat at least as high as the melting point of the API. It is believed that the present formulation will be particularly suited to formulations of terameprocol and paclitaxel.

The present formulations also lend themselves to combination products comprising water soluble drugs. Water soluble drugs such as methotrexate, docetaxel, and hydrocortisone sodium succinate are just a few examples of agents that may be added to the present formulation. The following methods and excipients are merely exemplary and are in no way limiting. The timing of the addition of the second agent will in part depend on its stability at the temperatures required to melt the insoluble drug into the cyclodextrin molecule. If unstable at elevated temperatures it is preferable to add the second agent after cooling the formulation.

## Claims

1. A formulation for solubilizing terameprocol, the formulation comprising a cyclodextrin and terameprocol with a sufficient amount of water to solubilize the cyclodextrin, and the formulation is obtainable by a method wherein the terameprocol is driven into the cyclodextrin by melting the terameprocol and said solubilized cyclodextrin is heated to a temperature which is above the melting point of terameprocol, but below the decomposition point of terameprocol.

2. The formulation of claim 1 wherein the formulation is free of organic solvents.

3. The formulation of claim 1 wherein terameprocoland the cyclodextrin are mixed in a ratio of from about 1:100 to about 1:10 respectively, on a percent weight basis.

4. The formulation of claims 1-3 wherein the cyclodextrin is selected from at least one of the following hydroxypropyl β- cyclodextrin, sufobutyl ether β- cyclodextrin.

5. The formulation of claim 4 wherein the cyclodextrin is hydroxypropyl β-cyclodextrin.

6. The formulation of claims 1-5 in which the formulation further comprises a second drug.

7. The formulation of claim 6 in which the second drug is also a water insoluble or poorly insoluble drug.

8. The formulation of claim 7 wherein the second drug is water soluble.

9. The formulation of claim 1 wherein the product is lyophilized.

10. The formulation of claim 1 wherein the pH is between 3 and 9.

11. The formulation of claim 10 wherein the pH is 7.1.

12. A process for making an aqueous pharmaceutical formulation from terameprocol, the process comprising dissolving a cyclodextrin in water, adding terameprocol, and heating the solution to a temperature above the melting point of terameprocol and below the decomposition temperature of the terameprocol.

13. A formulation according to claim 1 for use in a method of treatment.

14. A formulation according to claim 1 for use in a method of treating a disease selected from the groups consisting of cancer, psoriasis, hypertension, obesity, type I or type II diabetes, pain, Alzheimer's disease, amyotrophic lateral sclerosis, Parkinson's disease, dementia, stroke, inflammatory disease, premalignant neoplasia or dysplasia, human immunodeficiency viruses, human T-celllymphotropic virus, human papilloma virus, herpes simplex viruses, hepatitis B virus , Epstein-Barr virus , Varicella-zoster, adenovirus, parvovirus, and Jakob Creutzfeldt virus.

## Patentansprüche

1. Formulierung zum Solubilisieren von Terameprocol, wobei die Formulierung ein Cyclodextrin und Terameprocol mit einer ausreichenden Wassermenge, um das Cyclodextrin zu solubilisieren, umfasst und die Formulierung durch ein Verfahren erhältlich ist, worin das Terameprocol durch Schmelzen des Terameprocol in das Cyclodextrin getrieben wird und das solubilisierte Cyclodextrin auf eine Temperatur erhitzt wird, die über dem Schmelzpunkt von Terameprocol, aber unter dem Zersetzungspunkt von Terameprocol liegt.

2. Formulierung nach Anspruch 1, wobei die Formulierung frei von organischen Lösungsmitteln ist.

3. Formulierung nach Anspruch 1, worin Terameprocol und das Cyclodextrin in einem Verhältnis von jeweils etwa 1:100 bis etwa 1:10, bezogen auf das Gewicht, gemischt sind.

4. Formulierung nach einem der Ansprüche 1 bis 3, worin das Cyclodextrin aus zumindest einem der folgenden ausgewählt ist: Hydroxypropyl-β-cyclodextrin, Sulfobutylether-β-cyclodextrin.

5. Formulierung nach Anspruch 4, worin das Cyclodextrin Hydroxypropyl-β-cyclodextrin ist.

6. Formulierung nach einem der Ansprüche 1 bis 5, wobei die Formulierung weiters ein zweites Arzneimittel umfasst.

7. Formulierung nach Anspruch 6, worin das zweite Arzneimittel auch ein wasserunlösliches oder schlecht unlösliches Arzneimittel ist.

8. Formulierung nach Anspruch 7, worin das zweite Arzneimittel wasserlöslich ist.

9. Formulierung nach Anspruch 1, worin das Produkt lyophilisiert ist.

10. Formulierung nach Anspruch 1, worin der pH zwischen 3 und 9 beträgt.

11. Formulierung nach Anspruch 10, worin der pH 7,1 beträgt.

12. Verfahren zum Herstellen einer wässrigen pharmazeutischen Formulierung aus Terameprocol, wobei das Verfahren das Lösen eines Cyclodextrins in Wasser, das Zusetzen von Terameprocol und das Erhitzen der Lösung auf eine Temperatur über dem Schmelzpunkt von Terameprocol und unter der Zersetzungstemperatur des Terameprocol umfasst.

13. Formulierung nach Anspruch 1 zur Verwendung in einem Behandlungsverfahren.

14. Formulierung nach Anspruch 1 zur Verwendung in einem Verfahren zur Behandlung einer Krankheit, die aus der aus Krebs, Psoriasis, Bluthochdruck, Adipositas, Typ-I- oder Typ-II-Diabetes, Schmerz, Alzheimer-Krankheit, amyotropher Lateralsklerose, Parkinson-Krankheit, Demenz, Schlaganfall, Entzündungskrankheit, prämaligner Neoplasie oder Dysplasie, menschlichen Immunschwächeviren, menschlichem T-Zell-lymphotropem Virus, menschlichem Papillomavirus, Herpes-simplex-Viren, Hepatitis-B-Virus, Epstein-Barr-Virus, Varicella zoster, Adenovirus, Parvovirus und Jakob-Creutzfeldt-Virus bestehenden Gruppe ausgewählt ist.

## Revendications

1. Formulation pour solubiliser du terameprocol, la formulation comprenant une cyclodextrine et du terameprocol avec une quantité d'eau suffisante pour solubiliser la cyclodextrine, et la formulation pouvant être obtenue par un procédé dans lequel le terameprocol est amené dans la cyclodextrine par fusion du terameprocol et ladite cyclodextrine solubilisée est chauffée à une température qui est supérieure au point de fusion du terameprocol, mais inférieure au point de décomposition du terameprocol.

2. Formulation selon la revendication 1, dans laquelle la formulation ne comporte pas de solvants organiques.

3. Formulation selon la revendication 1, dans laquelle le terameprocol et la cyclodextrine sont mélangés selon un rapport d'environ 1:100 à environ 1:10, respectivement, sur une base de pourcentage en poids.

4. Formulation selon les revendications 1 à 3, dans laquelle la cyclodextrine est choisie parmi au moins l'un des éléments suivants : hydroxypropyl β-cyclodextrine, sufobutyl éther β-cyclodextrine.

5. Formulation selon la revendication 4, dans laquelle la cyclodextrine est l'hydroxypropyl 5-cyclodextrine.

6. Formulation selon les revendications 1 à 5, dans laquelle la formulation comprend en outre un second médicament.

7. Formulation selon la revendication 6, dans laquelle le second médicament est également un médicament insoluble ou faiblement insoluble dans l'eau.

8. Formulation selon la revendication 7, dans laquelle le second médicament est soluble dans l'eau.

9. Formulation selon la revendication 1, dans laquelle le produit est lyophilisé.

10. Formulation selon la revendication 1, dans laquelle le pH est compris entre 3 et 9.

11. Formulation selon la revendication 10, dans laquelle le pH est de 7,1.

12. Procédé de fabrication d'une formulation pharmaceutique aqueuse à partir de terameprocol, le procédé comprenant la dissolution d'une cyclodextrine dans de l'eau, l'ajout de terameprocol, et le chauffage de la solution à une température supérieure au point de fusion du terameprocol et inférieure à la température de décomposition du terameprocol.

13. Formulation selon la revendication 1 destinée à être utilisée dans un procédé de traitement.

14. Formulation selon la revendication 1 destinée à être utilisée dans un procédé de traitement d'une maladie choisie parmi le groupe constitué de cancer, psoriasis, hypertension, obésité, diabète de type I ou de type II, douleur, maladie d'Alzheimer, sclérose latérale amyotrophique, maladie de Parkinson, démence, accident vasculaire cérébral, maladie inflammatoire, néoplasie ou dysplasie précancéreuse, il virus de l'immunodéficience humaine, virus T-lymphotropique humain, virus du papillome humain, virus de l'herpès simplex, virus de l'hépatite B, virus d'Epstein-Barr, varicelle-zona, adénovirus, parvovirus, et virus de Creutzfeldt-Jakob.
